(19) Europäisches Patentamt
European Patent Office
Office européen des brevets

(11) Numéro de publication : **0 433 190 B1**

# (12) FASCICULE DE BREVET EUROPEEN

(45) Date de publication du fascicule du brevet : **21.04.93 Bulletin 93/16**

(51) Int. Cl.[5] : **C07C 57/13,** C07C 51/12

(21) Numéro de dépôt : **90420537.4**

(22) Date de dépôt : **11.12.90**

## (54) Procédé de préparation d'acides hexènedioiques-1,6.

(30) Priorité : **13.12.89 FR 8916754**

(43) Date de publication de la demande : **19.06.91 Bulletin 91/25**

(45) Mention de la délivrance du brevet : **21.04.93 Bulletin 93/16**

(84) Etats contractants désignés : **BE DE ES FR GB IT NL**

(56) Documents cités : **EP-A- 0 124 160**

(73) Titulaire : **RHONE-POULENC CHIMIE**
**25, quai Paul Doumer**
**F-92408 Courbevoie Cédex (FR)**

(72) Inventeur : **Denis, Philippe**
**16, Allée des Troenes**
**F-69150 Decines (FR)**

(74) Mandataire : **Vignally, Noel et al**
**Rhône-Poulenc Chimie Direction de la Propriété Industrielle Centre de Recherches des Carrières B.P. 62**
**F-69192 Saint-Fons Cédex (FR)**

EP 0 433 190 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation d'acides hexènedioïques-1,6. Par acide hexènedioïque-1,6 on entend plus particulièrement l'acide hexène-3 dioïque-1,6. L'acide hexène-3 dioïque-1,6 peut être hydrogéné en acide adipique.

L'acide adipique, l'une des matières premières du nylon 66 est produit avec de forts tonnages et de ce seul fait, toute nouvelle voie d'accès à ce diacide et/ou à ses dérivés présente un intérêt de principe immédiatement perceptible.

La présente invention a plus spécifiquement pour objet un procédé de préparation de l'acide hexène-3 dioïque-1,6 par réaction du monoxyde de carbone et de l'eau sur au moins un butène disubstitué par des groupes acyloxy en présence d'un catalyseur à base de palladium.

Il est indiqué dans le brevet américain n° 4,611,082 que la carbonylation d'une solution du diacétoxy-1,4 butène-2 dans un solvant aprotique, polaire et non basique choisi dans le groupe constitué par les nitriles, le bis(méthoxy-2) butène-2, le bis(méthoxy-2 éthyl)éther et le chlorure de méthylène à 80 - 140°C en présence d'un halogénure d'un métal de transition n'est pratiquement pas observée et qu'en présence d'un alcool les vitesses augmentent et sont comparables à celles constatées pour la carbonylation du butène-2 diol-1,4. A propos de ce dernier substrat mentionné il est également indiqué qu'il ne permet pas d'atteindre dans les conditions précitées des rendements satisfaisants en produits linéaires de carbonylation et, dans ce contexte, préférence est donnée aux substrats substitués en position 1,4 par des groupements alcoxy.

Dans ce contexte il apparaît donc que le diacétoxy-1,4 butène-2 ne peut être considéré comme un substrat prometteur pour former des produits linéaires dicarbonylés.

Dans la demande de brevet français n° 89/06018 il a été proposé un procédé de préparation d'acides hexènedioïques-1,6 par réaction du monoxyde de carbone et de l'eau sur au moins un butène disubstitué par des groupes acyloxy en présence d'un catalyseur à base de palladium et d'un chlorure d'onium quaternaire d'un élément du groupe VB choisi parmi l'azote et le phosphore, ledit élément étant tétracoordonné à des atomes de carbone, l'azote pouvant être coordoné à deux atomes de phosphore pentavalents.

Il a également été proposé dans cette demande de conduire ladite réaction dans la N-méthylpyrrolidone-2.

Le procédé en cause qui conduit à des résultats appréciables tant sur le plan de l'activité que sur celui de la sélectivité en produit dicarbonylé linéaire présente cependant l'inconvénient de requérir la présence d'au moins un chlorure d'onium quaternaire au sens indiqué ci-avant, promoteurs relativement onéreux ou peu disponibles et susceptibles de se dégrader lors d'un usage prolongé.

C'est pourquoi il peut s'avérer utile de proposer une alternative audit procédé par laquelle le promoteur organique chloré n'est plus indispensable et tout ou partie dudit promoteur peut être remplacée par un promoteur halogéné minéral, plus disponible et relativement plus stable lors d'un usage prolongé.

La présente invention a donc pour objet un procédé de préparation de d'acide hexène-3 dioïque par réaction du monoxyde de carbone et de l'eau sur au moins un butène disubstitué par des groupes acyloxy en présence d'un catalyseur à base de palladium et, le cas échéant d'un chlorure, caractérisé en ce que la réaction est conduite en présence d'un solvant polaire aprotique basique et également en présence d'au moins un halogénure minéral dont le cation est choisi parmi des métaux alcalins et les cations des métaux alcalino-terreux, l'anion halogénure étant choisi parmi le chlorure et le bromure.

Il a en effet été trouvé de manière tout à fait surprenante qu'un tel procédé permet de réaliser la dicarbonylation dans des conditions de pression et de température acceptables à l'échelle industrielle, avec une sélectivité appréciable en produit dicarbonylé linéaire.

Le procédé en cause peut être représenté par le schéma réactionnel suivant, lorsqu'on part d'un butène-2 disubstitué en 1,4 par des groupes acyloxy,

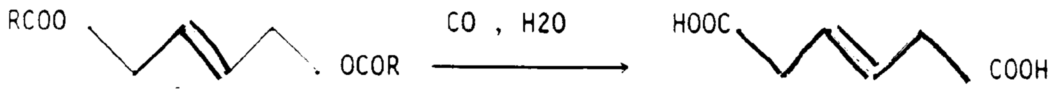


dans lequel R représente :

. un radical alkyle linéaire ou ramifié, contenant de 1 à 12 atomes de carbone, éventuellement substitué par un groupe phényle ;
ou
. un radical aryle contenant de 6 à 10 atomes de carbone, éventuellement substitué par un ou deux radicaux alkyles contenant de 1 à 4 atomes de carbone ;

et peut comporter de 1 à 3 substituants choisis parmi les atomes de fluor et de chlore et les groupements dialkylamino et N,N-dialkylamido dont les radicaux alkyles renferment au plus 4 atomes de carbone.

Le procédé, selon la présente invention, requiert la mise en oeuvre d'au moins un butène disubstitué par des groupes acyloxy. Par groupe acyloxy, on entend des groupes de formule RCOO- dans laquelle R a la signification donnée précédemment ; par butènes disubstitués on entend les composés du butène-2 substitués en positions 1 et 4 et les composés du butène-1 substitués en positions 3 et 4. Bien entendu des mélanges de butène-2 disubstitués par des groupes acyloxy de nature distincte, des mélanges de butène-1 disubstitués par des groupes acyloxy de nature distincte ou des mélanges de butène-2 et de butène-1 disubstitués peuvent être mis en oeuvre dans le cadre du présent procédé.

Il a en effet été constaté par la Demanderesse que la sélectivité en acide linéaire est sensiblement la même que l'on parte d'un butène-2 disubstitué par des groupes acyloxy en positions 1,4 ou d'un butène-1 disubstitué par des groupes acyloxy en positions 3 et 4.

A titre d'exemples de butènes disubstitués par des groupes acyloxy on peut citer : les diacétoxybutènes, les dipropionyloxybutènes, les dibutyryloxybutènes et les dibenzoyloxybutènes.

Le diacétoxy-1,4 butène-2, le diacétoxy-3,4 butène-1 et leurs mélanges conviennent plus particulièrement bien à la mise en oeuvre de la présente invention.

Le procédé selon la présente invention requiert également la présence d'eau.

La quantité d'eau à mettre en oeuvre dans le cadre du présent procédé n'est pas critique et peut varier dans de larges limites.

Pour une bonne mise en oeuvre de la réaction le rapport molaire de l'eau au butène disubstitué sera compris entre 1 et 100 et, de préférence entre 1 et 50.

Le procédé selon la présente invention est conduit en présence d'un catalyseur à base de palladium.

Bien que la nature précise de l'espèce (ou des espèces) catalytiquement active(s) dans la réaction en cause ne soit pas totalement élucidée, la Demanderesse a constaté que divers composés du palladium et le palladium métallique sont susceptibles d'être des précurseurs utiles dans la mise en oeuvre du présent procédé.

Parmi les sources de palladium pouvant être mises en oeuvre pour réaliser le procédé faisant l'objet de l'invention, on peut citer :

. le palladium métallique déposé le cas échéant sur un support, tel que le charbon, l'alumine, ou la silice,
. $PdCl_2$, $Pd(OAc)_2$, $PBu_4PdCl_3$(Bu = n- butyle)
. les sels ou les complexes $\pi$-allyliques de palladium, dont l'anion coordonné au cation Pd est choisi parmi les anions suivants: carboxylates tels que formiate, acétate, propionate, benzoate; acétylacétonate, halogénures tels que $Cl^-$ et $Br^-$ et de préférence $Cl^-$ ;

On recourt avantageusement au chlorure de palladium.

La quantité précise de catalyseur à mettre en oeuvre, qui peut varier dans de larges limites, dépendra avant tout d'un compromis entre l'efficacité souhaitée et la dépense en catalyseur et des autres conditions choisies pour la réaction. En général, de bons résultats peuvent être obtenus avec une concentration de palladium dans le milieu réactionnel, comprise entre $10^{-3}$ et 1 mol/l. De préférence, cette concentration est comprise entre $2.10^{-3}$ et $5.10^{-2}$ Mol/l.

L'une des caractéristiques essentielles du présent procédé réside dans le fait que la réaction est conduite en présence d'un solvant polaire, aprotique et basique.

Par solvant polaire, aprotique et basique, la Demanderesse entend les composés de formule (I) :

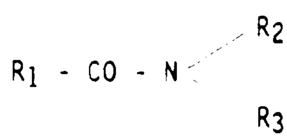

(I)

dans laquelle $R_1$, $R_2$ et $R_3$, identiques ou différents représentent un radical alkyle, un radical cycloalkyle, un radical aralkyle ou un radical aryle monocyclique ayant au plus 10 atomes de carbone, deux radicaux $R_1$, $R_2$ ou $R_3$, pouvant constituer ensemble un radical unique divalent
$-(CH_2)_y-$, y étant un entier compris entre 3 et 12, $R_1$ pouvant en outre représenter un radical



(II)

dans lequel $R_4$ et $R_5$, identiques ou différents représentent un radical alkyle ayant au maximum 4 atomes de carbone.

A titre d'exemple de tels solvants, on peut citer :

la tétraméthylurée, le N,N-diméthylacétamide, le N,N-diéthylacétamide, le N,N-dicyclohexylacétamide, le N,N-diméthylpropionamide, le N-N-diéthylpropionamide, le N,N-diéthyl-n-butyramide, le N,N-diméthylbenzamide, le N,N-dicyclohexylbenzamide, le N,N-diéthyl-m-toluamide, la N-acétylpyrrolidine, la n-acétylpipéridine, la N-(n-butyryl)pipéridine, la N-méthylpyrrolidone-2, la N-éthylpyrrolidone-2, la N-méthylpipéridone-2, le N-méthyl-epsiloncaprolactame.

La N-méthylpyrrolidone-2 convient particulièrement bien à la mise en oeuvre du présent procédé.

En général la quantité de solvant représente au moins 10% en volume du milieu réactionnel ; de bons résultats sont obtenus lorsqu'on en utilise de l'ordre de 20% à 85% en volume.

Bien entendu il est tout à fait possible d'utiliser un mélange de solvants du type précité ou un mélange d'au moins un tel solvant et d'un solvant inerte dans les conditions réactionnelles et n'entrant pas dans la catégorie précitée, tels une cétone, un hydrocarbure aliphatique saturé ou un hydrocarbure aromatique.

Selon une autre caractéristique du présent procédé, la réaction est conduite également en présence d'un halogénure dont le cation est choisi parmi les cations des métaux alcalins et les cations des métaux alcalino-terreux, l'anion halogénure étant choisi parmi le chlorure et le bromure.

A titre d'exemples de tels halogénures on peut citer : LiCl, LiBr et $CaCl_2$.

Il a été constaté que l'effet bénéfique apporté par la présence dans le milieu de carbonylation d'un chlorure ou bromure alcalin ou alcalino-terreux est sensible à partir d'un rapport molaire $Cl^-$(ou $Br^-$)/ palladium de 0,5 ; notamment un effet particulièrement intéressant a été constaté lorsque ledit rapport est compris entre 1 et 50, un rapport plus élevé, n'étant toutefois pas nocif pour la réaction.

Bien entendu, on peut mettre en oeuvre dans le cadre de la présente invention un mélange de tels halogénures minéraux ou un mélange d'au moins un halogénure d'onium quaternaire au sens rappelé en tête du présent mémoire.

On recourt avantageusement à un (ou des) chlorure(s).

La réaction pourra être généralement conduite en phase liquide à une température comprise entre 50 et 150°C, de préférence entre 80 et 130°C, sous une pression d'oxyde de carbone comprise entre 10 et 250 bar (1000 et 25 000 KPa), de préférence entre 15 et 180 bar (1500 et 18 000 KPa).

Des gaz inertes, tels l'azote, l'argon ou le gaz carbonique, peuvent être présents à côté de l'oxyde de carbone.

Selon une variante avantageuse du procédé selon la présente invention la réaction est conduite dans la N-méthylpyrrolidone.

En fin de réaction ou du temps de réaction voulu, on récupère le diacide recherché par tout moyen approprié, par exemple par extraction.

Les exemples ci-après illustrent l'invention.

Dans ces exemples, le taux de transformation est de 100% et on observe la formation des divers acides suivants :

- HD : mélange d'acides hexène-3 et hexène-2 dioïques dans lequel l'acide hexène-3 dioïque est majoritaire.
- PDO : acide pentadiènoïque
- IT : acide hydroxy-5 pentène-3 oïque

dont on indique pour chaque groupe le nombre de moles formées pour 100 moles de diacétoxybutène chargées.

EXEMPLES 1 A 8 ; Essais témoins (a) et (b) :

Dans un autoclave en acier inoxydable (HastelloyB2) de 125 cm3, préalablement purgé à l'argon, on introduit :

- 8,6 g (50 mmol) de diacétoxy-1,4 butène-2
- 1,8 g (100 mmol) d'eau
- 1 mat-g de palladium sous la forme indiquée dans le tableau ci-après :
- 17 mmol de chlorure minéral dont la nature est précisée dans le tableau précité
- 25 cm3 de N-méthylpyrrolidone-2

L'autoclave est fermé hermétiquement, placé dans un four agité et raccordé à l'alimentation de gaz sous pression. On purge le réacteur à froid avec de l'oxyde de carbone et on le porte à 100°C. On régule ensuite la pression à 120 bar. Après 6 heures de réaction (sauf indication contraire), l'autoclave est refroidi et dégazé.

Le mélange réactionnel résultant est dilué à 100 cm3 par le solvant.

Une partie aliquote est estérifiée par le méthanol puis analysée par chromatographie en phase gazeuse.

TABLEAU

--------

| Réf | Palladium | Chlorure | t(mn) | HD(%) | PDO (%) | IT (%) |
|---|---|---|---|---|---|---|
| a | $PdCl_2$ | Néant | 360 | 9 | 6 | 30 |
| 1 | $PdCl_2$ | LiCl | 60 | 59 | 4 | 9 |
| 2 | $PdCl_2$ | LiBr | 360* | 23 | 0 | 0 |
| 3 | $PdCl_2$ | KCl | 360* | 15 | 7,5 | 30 |
| 4 | $PdCl_2$ | NaCl | 360* | 21 | 8,5 | 28 |
| 5 | $PdCl_2$ | CsCl | 360* | 19 | 5,0 | 34 |
| 6 | $PdCl_2$ | $CaCl_2$ | 60 | 70 | 9 | 6,5 |
| b | $Pd(OAc)_2$ | Néant | 360 | 0 | 0 | 0 |
| 7 | $Pd(OAc)_2$ | LiCl | 60 | 17 | 25 | 18 |
| 8 | $PdCl_2$ | $PBu_4Cl$(**)<br>LiCl(**) | 360 | 69 | 4,5 | 0 |

(*) absorption non terminée

(**) 8,5 mmol

## Revendications

1. Procédé de préparation d'acides hexènedioïques-1,6 par réaction de l'oxyde de carbone et de l'eau sur au moins un butène disubstitué par des groupes acyloxy en présence d'un catalyseur à base de palladium et, le cas échéant d'un chlorure, caractérisé en ce que la réaction est conduite en présence d'un solvant polaire, aprotique et basique et en présence d'au moins un halogénure minéral dont le cation est choisi parmi les cations des métaux alcalins et les cations des métaux alcalino-terreux et dont l'anion est choisi parmi le chlorure et le bromure.

2. Procédé selon la revendication 1, caractérisé en ce que le solvant est choisi parmi les composés de formule (I) :

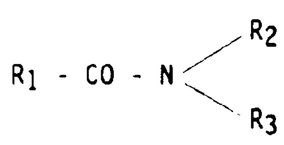

(I)

dans laquelle $R_1$, $R_2$ et $R_3$, identiques ou différents représentent un radical alkyle, un radical cycloalkyle,

un radical aralkyle ou un radical aryle monocyclique ayant au plus 10 atomes de carbone, deux radicaux $R_1$, $R_2$ ou $R_3$, pouvant constituer ensemble un radical unique divalent - $(CH_2)_y$ -, y étant un entier compris entre 3 et 12, $R_1$ pouvant en outre représenter un radical

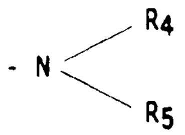

(II)

dans lequel $R_4$ et $R_5$, identiques ou différents représentent un radical alkyle ayant au maximum 4 atomes de carbone.

3. Procédé selon la revendication 1 ou 2 caractérisé ce que la quantité de solvant représente au moins 10% en volume du milieu réactionnel.

4. Procédé selon l'une quelconque des revendications précédentes caractérisé en ce que le solvant est la N-méthylpyrrolidone-2.

5. Procédé selon l'une quelconque des revendications précédentes caractérisé en ce que le rapport molaire de l'anion chlorure (ou bromure) au palladium est compris entre 1 et 50.

6. Procédé selon l'une quelconque des revendications précédentes, caractérisé en ce que la concentration du palladium dans le milieu réactionnel est compris entre $10^{-3}$ et 1 mol/l.

7. Procédé selon l'une quelconque des revendications précédentes, caractérisé en ce que le rapport molaire de l'eau au butène disubstitué est compris entre 1 et 100 et, de préférence, entre 1 et 50.

8. Procédé selon l'une quelconque des revendications précédentes, caractérisé en ce que la température de réaction est comprise entre 50 et 150°C et, de préférence, entre 80 et 130°C.

9. Procédé selon l'une quelconque des revendications précédentes, caractérisé en ce que la pression est comprise entre 10 et 250 bar (1000 et 25 000 KPa) et, de préférence, entre 15 et 180 bar (1500 et 18 000 KPa).

10. Procédé selon l'une quelconque des revendications précédentes, caractérisé en ce que le butène disubstitué est choisi parmi le diacétoxy-1,4 butène-2 et le diacétoxy-3,4 butène-1 et leurs mélanges.

11. Procédé selon l'une quelconque des revendications précédentes, caractérisé en ce que l'halogénure minéral est un chlorure.

12. Procédé selon l'une quelconque des revendications précédentes, caractérisé en ce que le palladium est engagé sous forme de chlorure de palladium.

**Patentansprüche**

1. Verfahren zur Herstellung von Hexen-1,6-di säuren durch Reaktion von Kohlenmonoxid und Wasser mit mindestens einem Buten, disubstituiert durch Acyloxy-Gruppen, in Anwesenheit eines Katalysators auf der Basis von Palladium und gegebenenfalls einem Chlorid, dadurch gekennzeichnet, daß die Reaktion in Anwesenheit eines basischen, aprotischen polaren Lösungsmittels und in Anwesenheit von mindestens einem Mineralhalogenid durchgeführt wird, dessen Kation unter den Alkalimetall-Kationen und den Erdalkalimetall-Kationen und dessen Anion unter Chlorid und Bromid ausgewählt werden.

2. Verfahren nach Anspruch 1, dadurch gekennzeichnet, daß das Lösungsmittel ausgewählt wird unter den Verbindungen der Formel (I)

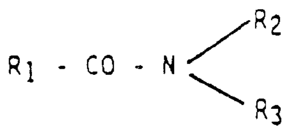

(I)

in der $R_1$, $R_2$ und $R_3$, gleich oder verschieden, einen Alkyl-Rest, einen Cycloalkyl-Rest, einen Aralkyl-Rest oder einen monocyclischen Aryl-Rest mit höchstens 10 Kohlenstoffatomen darstellen, wobei zwei Reste $R_1$, $R_2$ oder $R_3$ zusammen einen einzigen divalenten Rest $-(CH_2)_y-$ bilden können, und y eine ganze Zahl zwischen 3 und 12 ist, wobei $R_1$ außerdem einen Rest (II)

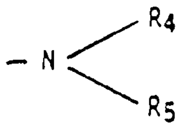

(II)

bedeuten kann, in dem $R_4$ und $R_5$, gleich oder verschieden, einen Alkyl-Rest mit höchstens 4 Kohlenstoffatomen darstellen.

3. Verfahren nach Anspruch 1 oder 2, dadurch gekennzeichnet, daß die Menge des Lösungsmittels mindestens 10 Volumenprozent des Reaktionsmediums beträgt.

4. Verfahren nach irgendeinem der vorstehenden Ansprüche, dadurch gekennzeichnet, daß das Lösungsmittel N-Methyl-2-pyrrolidon ist.

5. Verfahren nach irgendeinem der vorstehenden Ansprüche, dadurch gekennzeichnet, daß das molare Verhältnis von Chlorid-(oder Bromid-)anion zu Palladium zwischen 1 und 50 beträgt.

6. Verfahren nach irgendeinem der vorstehenden Ansprüche, dadurch gekennzeichnet, daß die Konzentration des Palladiums in dem Reaktionsmedium zwischen $10^{-3}$ und 1 Mol/l beträgt.

7. Verfahren nach irgendeinem der vorstehenden Ansprüche, dadurch gekennzeichnet, daß das molare Verhältnis von Wasser zu disubstituiertem Buten zwischen 1 und 100, vorzugsweise zwischen 1 und 50, beträgt.

8. Verfahren nach irgendeinem der vorstehenden Ansprüche, dadurch gekennzeichnet, daß die Temperatur der Reaktion zwischen 50 °C und 150 °C, vorzugsweise zwischen 80 °C und 130 °C, beträgt.

9. Verfahren nach irgendeinem der vorstehenden Ansprüche, dadurch gekennzeichnet, daß der Druck zwischen 10 bar und 250 bar (1000 und 25000 kPa), vorzugsweise zwischen 15 bar und 180 bar (1500 und 18000 kPa), beträgt.

10. Verfahren nach irgendeinem der vorstehenden Ansprüche, dadurch gekennzeichnet, daß das disubstituierte Buten ausgewählt wird unter 1,4-Diacetoxy-2-buten und 3,4-Diacetoxy-1-buten sowie deren Mischungen.

11. Verfahren nach irgendeinem der vorstehenden Ansprüche, dadurch gekennzeichnet, daß das Mineralhalogenid ein Chlorid ist.

12. Verfahren nach irgendeinem der vorstehenden Ansprüche, dadurch gekennzeichnet, daß das Palladium in Form von Palladiumchlorid eingesetzt wird.

## Claims

1. Process for the preparation of 1,6-hexenedioic acids by reaction of carbon monoxide and water with at least one butene disubstituted by acyloxy groups in the presence of a catalyst based on palladium and, if appropriate, a chloride, characterised in that the reaction is conducted in the presence of an aprotic

and basic polar solvent and in the presence of at least one inorganic halide whose cation is chosen from alkali metal cations and alkaline-earth metal cations and whose anion is chosen from chloride and bromide.

2. Process according to Claim 1, characterised in that the solvent is chosen from the compounds of formula (I) :

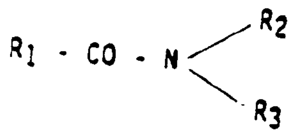

(I)

in which $R_1$, $R_2$ and $R_3$, which are identical or different, denote an alkyl radical, a cycloalkyl radical, an aralkyl radical or an aryl radical, monocyclic, containing not more than 10 carbon atoms, it being possible for two radicals $R_1$, $R_2$ or $R_3$ to form together a single divalent radical $-(CH_2)_y-$, y being an integer between 3 and 12, it being additionally possible for $R_1$ to denote a radical

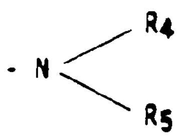

(II)

in which $R_4$ and $R_5$, which are identical or different, denote an alkyl radical containing not more than 4 carbon atoms.

3. Process according to Claim 1 or 2, characterised in that the quantity of solvent represents at least 10 % by volume of the reaction mixture.

4. Process according to any one of the preceding claims, characterised in that the solvent is N-methyl-2-pyrrolidone.

5. Process according to any one of the preceding claims, characterised in that the molar ratio of the chloride (or bromide) anion to palladium is between 1 and 50.

6. Process according to any one of the preceding claims, characterised in that the concentration of palladium in the reaction mixture is between $10^{-3}$ and 1 mol/l.

7. Process according to any one of the preceding claims, characterised in that the molar ratio of water to the disubstituted butene is between 1 and 100 and, preferably, between 1 and 50.

8. Process according to any one of the preceding claims, characterised in that the reaction temperature is between 50 and 150°C and, preferably, between 80 and 130°C.

9. Process according to any one of the preceding claims, characterised in that the pressure is between 10 and 250 bar (1000 and 25,000 kPa) and, preferably, between 15 and 180 bar (1500 and 18,000 kPa).

10. Process according to any one of the preceding claims, characterised in that the disubstituted butene is chosen from 1,4-diacetoxy-2-butene and 3,4-diacetoxy-1-butene and mixtures thereof.

11. Process according to any one of the preceding claims, characterised in that the inorganic halide is a chloride.

12. Process according to any one of the preceding claims, characterised in that the palladium is introduced in the form of palladium chloride.